# FASCICULE DE BREVET EUROPEEN

(11) **EP 0 904 390 B1**
(45) Date de publication et mention de la délivrance du brevet: **05.09.2007**
(21) Numéro de dépôt: 97926061.9
(22) Date de dépôt: 30.05.1997
(51) Int. Cl.: C12N 15/82, C12N 5/04, C07K 14/415

(54) **GENOME VEGETAL RECOMBINE, COMPORTANT DES GENES SPECIFIQUES DES CHICOREES ET UNE SEQUENCE NUCLEOTIDIQUE CONFERANT UNE STERILITE MALE ET SON UTILISATION**
PFLANZENGENOM ENTHALTEND CHICOREE-GENE UND EINE DNS KODIEREND FUER MAENNLICHE STERILITAET UND SEINE VERWENDUNG
RECOMBINANT PLANT GENOME, COMPRISING SPECIFIC CHICORY GENES AND A NUCLEOTIDE SEQUENCE CONFERRING MALE STERILITY, AND ITS USE.

(30) Priorité: 31.05.1996 FR 9606725
(43) Date de publication de la demande: 31.03.1999
(73) Titulaire: Florimond Desprez Veuve et Fils, 59242 Capelle-en-Pévèle (FR)
(72) Inventeur: DELESALLE, Louis, F-59242 Cappelle-en-Pévèle (FR); DHELLEMMES, Charles, F-59242 Cappelle-en-Pévèle (FR); DESPREZ, Michel, F-59242 Cappelle-en-Pévèle (FR)
(74) Mandataire: Warcoin, Jacques
(86) Numéro de dépôt international: PCT/FR1997/000944
(87) Numéro de publication internationale: WO 1997/045548

(56) Documents cités:
- EP-A- 0 771 523
- GB-A- 2 211 205
- COMPTES RENDUS DE L'ACADEMIE D'AGRICULTURE DE FRANCE, vol. 80, no. 7, 1994, PARIS FR, pages 63-67, XP000197041 C. RAMBAUD ET AL.: "Chicory-sunflower protoplast fusion"
- THEORETICAL AND APPLIED GENETICS, vol. 87, no. 3, 1993, BERLIN DE, pages 347-352, XP000197036 C RAMBAUD ET AL.: "Male-sterile chicory cybrids obtained by intergeneric protoplast fusion"
- MOLECULAR AND GENERAL GENETICS, vol. 227, no. 3, Juillet 1991, BERLIN DE, pages 369-376, XP000608949 R.H. KÖHLER ET AL: "CYTOPLASMIC MALE STERILITY IN SUNFLOWER IS CORRELATED WITH THE CO-TRANSCRIPTION OF A NEW OPEN READING FRAME WITH THE ATPA GENE" cité dans la demande

### LISTE DE SEQUENCES

(1) INFORMATIONS GENERALES:
   (i) DEPOSANT:
      (A) NOM: FLORIMOND DESPREZ VEUVE ET FILS
      (B) RUE: BP 41
      (C) VILLE: CAPPELLE EN PEVELE
      (E) PAYS: PARIS
      (F) CODE POSTAL: 59242
   (ii) TITRE DE L' INVENTION: GENOME VEGETAL RECOMBINE, MITOCHONDRIE ET CELLULE LE CONTENANT ET PROCEDE DE SELECTION D'UNE STERILITE MALE CYTOPLASMIQUE CHEZ UNE PLANTE DU GENRE CICHORIUM
   (iii) NOMBRE DE SEQUENCES: 2
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9606725
      (B) DATE DE DEPOT: 31-MAI-1996
(2) INFORMATIONS POUR LA SEQ ID NO: 1:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
      CCCCCTCCCT GGTGGATCCG GCG 23
(2) INFORMATIONS POUR LA SEQ ID NO: 2:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 2:
      CCCTCTATGA GTACCGTTCT CTCACG 26

## Description

La présente invention se rapporte a l'utilisation de séquences nucléotidiques permettant de conférer- une stérilité mâle de type cytoplasmique à des plantes du genre Cichorium.

Le genre Cichorium rassemble des plantes présentant un grand intérêt agro-alimentaire, comme les divers types de chicorée ou les endives.

Il s'agit de plantes possédant à la fois des systèmes de reproduction mâle et femelle, et qui ont donc une capacité à s'autoféconder. Un tel évènement est indésirable lorsque l'on souhaite effectuer des croisements avec une plante d'une autre variété, en vue de l'obtention d'hybrides.

Chez Cichorium intybus, des plantes à stérilité mâle nucléaire ont déjà été proposées. Cependant la stérilité mâle cytoplasmique constitue une solution d'intérêt pour la production d'espèces hybrides.

La stérilité mâle cytoplasmique est une caractéristique transmise par le parent femelle d'une plante (hérédité maternelle), et qui prévient la formation de pollen viable. Une stérilité mâle de bonne qualité ne doit pas affecter la fertilité femelle de la plante, pour permettre son croisement avec des plantes mâles fertiles.

Rambaud et al, C.R. Acad. Agric. Fr., 80(7), 1994 porte sur des résultats préliminaires d'une fusion de protoplantes chicorée/tournesol en vue de préparer des chicorées mâles stériles cytoplasmiques.

Il est donc souhaitable de pouvoir disposer d'un système conférant une telle stérilité mâle cytoplasmique, qui soit stable.

Il est également souhaitable de disposer d'un marqueur fiable de cette stérilité mâle cytoplasmique permettant la sélection des cellules végétales avant même le développement d'une plante complète présentant l'ensemble des caractères phénotypiques.

C'est pourquoi la présente invention a pour objet un génome végétal recombiné, caractérisé en ce qu'il comporte des gènes spécifiques des chicorées et une séquence nucléotidique conférant une stérilité mâle, portée par la séquence orf 522 du tournesol délimitée par les amorces des séquences SEQ ID N°1 et 2, ou par une séquence présentant au moins 90% d'homologie avec ladite séquence orf 522 délimitée par les amorces des séquences SEQ ID N°1 et 2.

La séquence orf 522 est une séquence qui a été mise en évidence chez le Tournesol (Helianthus), notamment H. annuus, où elle semble associée à une stérilité mâle cytoplasmique (Köhler et al, Mol. Gen. Genet., 1991, 227: 369-376).

La Demanderesse a trouvé que la présence de cette séquence orf 522 dans le génome d'une plante du genre Cichorium était liée à une stérilité mâle cytoplasmique.

Des séquences convenant à la mise en oeuvre de l'invention comprennent des séquences portées par la séquence orf 522 telle qu'elle a été précédemment décrite. Des séquences appropriées présentent avantageusement 90% de similarité avec ladite séquence orf 522 délimitée par les amorces des séquences SEQ ID N°1 et 2, et comprennent en particulier les séquences codant pour la même protéine, compte tenu de la dégénérescence du code génétique, ou pour une protéine dans laquelle certains acides aminés ont été remplacés par des acides aminés équivalents. Par acides aminés équivalents, on entend des acides aminés présentant un comportement chimique analogue et/ou des poids moléculaires voisins. Elles comprennent également les séquences codant pour une protéine dans laquelle un ou plusieurs acides aminés non essentiels à l'activité ont été délétés ou remplacés.

Le génome peut être de type nucléaire ou mirochondrial.

Lorsque la séquence est présente dans le génome nucléaire, celui-ci comportera en outre une préséquence permettant l'importation dans les mitochondries du produit de traduction de ladite séquence.

De préférence, le génome recombiné est un génome mitochondrial. L'invention a donc également pour objet une mitochondrie comportant un génome recombiné tel que défini précédemment.

En particulier, l'invention a pour objet une mitochondrie caractérisée en ce qu'elle contient au moins un fragment nucléotidique de 347 pb, portée par la séquence orf 522 delimité par les amorces des séquences SEQ ID N°1 et 2, ou une séquence présentant au moins 90% d'homologie avec ledit fragment délimité par les amorces des séquences SEQ ID N°1 et 2 .

la séquence de 347 pb est délimitée, à l'intérieur de la séquence orf 522 (Köhler et al, Mol. Gen. Genet 1991), par les amorces de séquences :
SEQ ID NO 1 : 5'CCCCCTCCCTGGTGGATCCGGCG 3'
SEQ ID NO 2 : 5'CCCTCTATGAGTACCGTTCTCTCACG 3'

L'invention concerne un cytoplasme végétal recombiné, caractérisé en ce qu'il contient un noyau comportant le génome du genre Cichorium, et un génome recombiné défini ci-dessus, en particulier un cytoplasme qui contient des mitochondries comportant une séquence nucléotidique portée par la séquence orf 522 délimitée par les amorces des séquences SEQ ID N°1 et 2 de Helianthus annuus, ou par une séquence présentant au moins 90% d'homologie délimitée par les amorces des séquences SEQ ID N°1 et 2.

Des cellules végétales recombinées contenant un tel cytoplasme sont comprises dans l'invention, et notamment une cellule végétale qui contient un noyau comportant substantiellement le génome d'une espèce choisie parmi Cichorium intybus et Cichorium endivia.

Parmi ces espèces, on peut citer, sans aucunement y limiter l'invention, les cultigroupes suivants :
Cichorium intybus L :
   - chicorées "sauvages améliorées"
   - chicorées "Barbe de Capucin"
   - chicorées "Pain de Sucre"
   - chicorées "Chioggia"
   - chicorées "Vérone"
   - chicorées "Catalogne"
   - chicorées "Trévise"
   - chicorées "Variegato di Castelfranco"
   - chicorées "Witloof" (ou "de Bruxelles" ou "à chicon")
   - chicorées "Soncino" chicorées "Industrielles" (à torréfier et à sucres)
   - chicorées "fourragères" ou "à gibiers"...
Cichorium endivia L :
   - chicorées "scaroles"
   - chicorées "frisées"...

Ce sont des chicorées dites à grosses racines (industrielles fourragères et Witloof) ou des chicorées dites à salade (vertes, rouges, panachées, à forcer ou non).

L'homme du métier pourra adapter sans difficulté l'invention, notamment en se référant à "Génétique et Amélioration de la Chicorée industrielle", DESPREZ et al., Séance spécialisée du 30 novembre 1994 à l'Académie d'Agriculture de France n° 80 (7) 48-49.

Selon un autre aspect, l'invention se rapporte à un procédé pour produire une plante du genre des Chicorées ou du matériel de reproduction de cette plante, présentant une stérilité mâle cytoplasmique, caractérisé en ce qu'on intègre dans le génome cellulaire de cette plante une séquence nucléotidique portée par la séquence orf 522 du tournesol délimitée par les amorces des séquences SEQ ID N°1 et 2, ou par une séquence présentant au moins 90% d'homologie avec ladite séquence orf 522 délimitée par les amorces des séquences SEQ ID N°1 et 2. La plante (ou le matériel de reproduction), à l'exception des variétés végétales, est comprise dans l'invention.

Elle concerne également un procédé essentiellement non biologique de préparation de plantes hybrides, caractérisé en ce qu'on croise une plante susceptible d'être obtenue par le procédé décrit ci-dessus, avec un plante de la même espèce, dépourvue de la séquence orf 522 de Helianthus annuus, où d'une séquence présentant au moins 90% d'homologie avec ladite séquence orf 522.

Selon encore un autre aspect, l'invention concerne un procédé de sélection d'une stérilité mâle cytoplasmique chez une plante du genre Cichorium, caractérisé en ce qu'on met en contact l'acide nucléique mitochondrial de la plante avec une sonde marquée comprenant au moins 10 nucléotides de la séquence orf 522 délimitée par les amorces des séquences SEQ ID N°1 et 2.

D'autres variantes et caractéristiques de l'invention apparaîtront à la lecture des exemples qui suivent.

### Exemple 1 : Préparation du matériel végétal

Des graines de Cichorium intybus L cv Pévèle ont été fournies par les Etablissements Florimond Desprez et des graines de Helianthus annuus (à stérilité mâle cytoplasmique ou SMC) ont été achetées dans le commerce.

Les graines de Cichorium intybus ont été stérilisées en surface avec une solution à 0,1% (p/v) de HgCl₂, lavées trois fois dans de l'eau distillée et placées dans des boites de Pétri sur un milieu de culture Heller (1953) (sels majeurs et mineurs exempts de FeCl₃) supplémentées avec 19,5 mg/l de Fe-EDTA 20 g/l de saccharose et 6 g/l d'Agar (Bioakar type E) et cultivées dans les conditions de culture décrites par Rambaud et al (1990). Les semis aseptiques ont été ensuite transferés dans des tubes de culture sur le même milieu. Les semences de Helianthus annuus ont été stérilisées avec une solution d'hypochlorure de calcium à 50 g/l, puis lavées trois fois dans de l'eau distillée et transférées dans une solution saccharose (10 g/l) / agar (0,6%).

Les feuilles de chicorée ont été récoltées sur des plantes de 12 à 14 jours. Les feuilles ont été coupées en morceaux et incubées dans une solution contenant 15 g/l de caylase 345, 0,5 g/l de caylase M2 (Cayla, Toulouse, France) et 90 g/l de mannitol.

Pour les semis de tournesol, les hypocotyles ont été prélevés 6 à 10 jours après la germination et incubés dans la même solution de macération.

Les protoplastes ont été incubés pendant 5 h 30 min. à 30° C dans l'obscurité sans agitation, purifiés par filtration à travers des tamis à mailles de 50 µm, récoltés et lavés trois fois par centrifugation à basse vitesse (100 x g) pendant 15 min.

Après l'élimination de la couche supérieure avec une pipette Pasteur, les protoplastes ont été mélangés dans le rapport 1:3 (tournesol/chicorée) afin d'obtenir une suspension contenant entre 7 et 11.10⁶ protoplastes/ml.

Les protoplastes ont été fusionnés selon la méthode de Kao (Wetter LRL and Constabel F (eds) Plant Tissue Culture Methods, ch 7, pp 49-56, 1982) avec les modifications suivantes : un volume d'une solution de mélange de protoplastes a été placé dans une boîte de Pétri et trois volumes de solution à 30% de polyéthylène glycol (PEG 4000 Serva) avec 10% de diméthylsulfoxide (DMSO) ont été ajoutés goutte à goutte. Après une homogénisation douce, on a laissé la solution reposer pendant trois minutes. Une minute plus tard on ajoute à nouveau 3,5 volumes de la solution numéro 3 de Kao (1982). Puis 3,5 volumes de solution numéro 3 de Kao (1982) sont ajoutés et 3 min plus tard 6 x 3,5 volumes de milieu de lavage (Saksi N et al. CR Acad. Sci. Paris 302 : 165-170, 1986) sont ajoutés. On récupère les protoplastes après 10-15 min, par centrifugation (8 min, 100 x g) ; ils sont ensuite lavés 3 fois par des aliquots de 8 ml du milieu de lavage et remis en suspension dans le milieu de culture MC1 (Saksi et al, 1986) dans lequel la concentration d'acide 1-naphthylacétique (NAA) est de 2 mg/l, celle de l'inositol 250 mg/l et de KNO₃ 144 mg/l.

Après un ou deux jours de culture dans ce milieu à une densité de 2.10⁴ protoplastes/ml, les hétérokaryocytes isolés sont cultivés à basse densité (12/100 µl) à 30° C dans un milieu MC1 modifié (0,5 mg/l NAA) auxquels sont ajoutés de l'acide 2-(N-morpholino)éthanesulfonique (MES) (5 mM), de l'hydrolysat de caséine (150 mg/l) et du lait de coco (2%; v/v).

Un mois plus tard, les colonies dérivées des fusions de protoplastes hétéroplasmiques sont transférées sur un milieu de prolifération puis sur un milieu de regénération (Rambaud et al. 1990). Après enracinement, les plantes ont été transférées en serre pour quelques semaines puis transplantées dans les champs.

Les plantes obtenues présentent un phénotype de chicorée. Parmi celles-ci, la lignée désignée par CT 52/3 présente une stérilité mâle par défaut de déhiscence des anthères ; la lignée CT 41/1 présente une stérilité mâle par absence complète d'anthère.

### Exemple 2

### Matériels et méthodes

### Matériel végétal

Seize populations de chicorées industrielles à cytoplasme normal numérotées FDI à FD16, la chicorée industrielle cv. Pévèle, et une famille de chicorée industrielle (CT 41/1) 20540 U à stérilité mâle cytoplasmique (SMC) fournies par la société Florimond-Desprez, une chicorée CT 52/3 (SMC), le tournesol cv. Mirasol (SMC). Les jeunes feuilles ont été prélevées sur des plantules élevées en serre et conservées à -80° C.

### ADN

Adaptation des protocoles du Laboratoire de Dellaporta (Plant. Mol. Report., 1983) pour la miniextraction d'ADN total : les opérations ont lieu à 4° C. Broyer finement avec de l'azote liquide un fragment foliaire de 150 à 200 mg en poids frais. Transférer dans un microtube de 2 ml. Ajouter 940 µl de solution tampon Tris-HCl 0,1 M pH 8,0, EDTA 50 mM, NaCl 0,5 M, β-mercaptoéthanol 10 mM. Ajouter 62 µl de SDS 20% et vortexer vigoureusement. Incuber à 65° C pendant 15 min. Ajouter 310 µl d'acétate de potassium 5 M et vortexer vigoureusement. Laisser précipiter pendant 30 min. Centrifuger. Centrifuger 15 min à 17.500 g. Transférer 1 ml du surnageant dans un microtube de 2 ml. Ajouter 0,5 ml d'isopropanol et mélanger. Laisser précipiter pendant 15 min. Centrifuger 10 min à 12.000 g. Décanter le surnageant avec une micropipette. Sécher les culots dans un appareil Speed-Vac pendant 10 min en position basse température. Redissoudre les culots dans 100 µl de solution tampon Tris-HCl 50 mM pH 8,0, EDTA 10 mM, ribonucléase A 0,2 mg/ml et incuber 2h à 37° C. Réaliser trois extractions phénoliques suivies d'une extraction au chloroforme. Ajouter 0,1 volume d'acétate de sodium 3 M pH 5,2 et deux volumes d'éthanol absolu à 4° C. Laisser précipiter 15 min à 4° C. Centrifuger 10 min à 12.000 g. Rincer le culot avec de l'éthanol 70% à 4° C. Centrifuger 3 min à 12.000 g. Décanter avec une micropipette et sécher au Speed-Vac pendant 10 min à basse température. Reprendre le culot dans une solution tampon Tris-HCl 1 mM pH 8,0, EDTA 0,1 mM.

Quantification à partir d'un aliquot de 5 µl par électrophorèse horizontale en gel d'agarose à 0,8%, TBE 1x, BET 0,5 µg/ml.

### PCR

Deux amorces de 23 et 26 bases qui délimitent un fragment de 347 pb interne à la séquence orf 522 (Köhler et al., Mol. Gen. Genet., 1991). Séquences des amorces :
SEQ ID NO 1: 5'CCCCCTCCCTGGTGGATCCGGCG 3'
SEQ ID NO 2 : 5'CCCTCTATGAGTACCGTTCTCTCACG3'

Progammation du thermocycleur 60 Bio-Med : premier cycle : 3 min, 92° C ; 30 cycles : 1.30s, 92° C ; 2.30s, 55° C ; 3.1 min, 72° C ; dernier cycle : 5 min, 72° C. Milieu réactionnel : Tampon Appligène 1x : Tris-HCl 10 mM pH 9.0. Triton X-100 0,1%, MgCl₂ 1,5mM, BSA 0,2 mg/ml ; dNTP 100 µM ; amorces 0,2 µM chacune ; Taq polymérase Appligène 2 U/100 µl ; ADN matrice 50 ng/ 100 µl ; H₂O QSP 10 µl ; huile minérale : 50 µl. Analyse des produits par électrophorèse horizontale en gel d'agarose 1,6% TBE lx, BET 0,5 µg/ml.

### Hybridations

Technique classique de transfert par la méthode de Southern ("Maniatis"). Marquage chimique de la sonde orf 522 avec le kit Dig-High-Prime (Böehringer-Mannheim). Révélation selon le protocole Böehringer-Mannheim avec le CSPD (Tropix) comme substrat chimioluminescent. Durée d'exposition des membranes : de une heure à une nuit pour les fragments très faiblement amplifiés.

### Résultats

Les ADN totaux de 4 individus par population FD1 à FD16, de 8 individus de la famille 20540 U, de la chicorée CT 52/3 et du tournesol "Mirasol", ont été extraits, soit au total 73 individus. L'absence d'inhibition de la PCR par des impuretés présentes dans les extraits d'ADN a été vérifiée en ajoutant quelques dizaines de femtogrammes du fragment de 347 pb à chaque milieu réactionnel. Les analyses PCR de l'ADN 52/3, de l'ADN tournesol "Mirasol", et des ADN de la famille 20540 U permettent toutes l'amplification d'une quantité importante (200 ng approx.) du fragment de 350 pb environ de l'orf 522. Ce fragment est absent chez toutes les chicorées à cytoplasme normal. L'analyse de ces produits PCR par hybridation moléculaire à l'aide d'une sonde orf 522 préparée par PCR à partir d'ADN total de tournesol Mirasol confirme l'homologie entre le fragment amplifié chez les chicorées (SMC) et la sonde.

### Conclusion

Les résultats obtenus ont permis de montrer que l'orf 522 n'est pas présente chez les chicorées fertiles. La détermination par PCR de la présence/absence de la séquence orf 522 peut donc être envisagée pour l'analyse de routine.

## Revendications

1. Génome végétal recombiné du genre Cichorium, **caractérisé en ce qu'**il comporte des gènes spécifiques des chicorées et une séquence nucléotidique de 347 pb dérivée de la séquence de l'orf 522 de tournesol (Heliantus annuus) et délimitée par les amorces de séquences SEQ ID No 1 et SEQ ID No 2 ou une séquence présentant au moins 90 % d'homologie avec ladite séquence nucléotidique de 347 pb dérivée de la séquence de l'orf 522 délimitée par les amorces des séquences SEQ ID N°1 et 2.

2. Mitochondrie comportant un génome recombiné selon la revendication 1.

3. Cytoplasme végétal comportant le génome du genre Cichorium et des mitochondries selon la revendication 2.

4. Cellule végétale recombinée comprenant un cytoplasme selon la revendication 3.

5. Procédé pour produire une plante du genre Cichorium présentant une stérilité mâle cytoplasmique, **caractérisé en ce qu'**on intègre dans le génome cellulaire de cette plante une séquence nucléotidique de 347 pb dérivée de la séquence de l'orf 522 de tournesol (Heliantus annuus) et délimitée par les amorces de séquences SEQ ID No 1 et SEQ ID No 2 ou une séquence présentant au moins 90 % d'homologie avec ladite séquence nucléotidique de 347 pb dérivée de la séquence de l'orf 522 délimitée par les amorces des séquences SEQ ID N°1 et 2.

6. Utilisation de la séquence nucléotidique de 347 pb dérivée de la séquence de l'orf 522 de tournesol (Heliantus annuus) et délimitée par les amorces de séquences SEQ ID No 1 et SEQ ID No 2 ou une séquence présentant au moins 90 % d'homologie avec ladite séquence nucléotidique de 347 pb dérivée de la séquence de l'orf 522 délimitée par les amorces des séquences SEQ ID N°1 et 2 pour conférer une stérilité mâle cytoplasmique à une plante du genre Cichorium.

7. Procédé de sélection d'une stérilité mâle cytoplasmique chez une plante du genre Cichorium **caractérisé en ce que** l'on met en contact l'acide nucléique mitochondrial de la plante avec une sonde marquée comprenant au moins 10 nucléotides consécutifs de la séquence nucléotidique de 347 pb dérivée de la séquence de l'orf 522 de tournesol (Heliantus annuus) et délimitée par les amorces de séquences SEQ ID No 1 et SEQ ID No 2.

## Claims

1. Recombinant plant genome of the genus Cichorium, **characterized in that** it comprises specific chicory genes and a 347 bp nucleotide sequence which is derived from the orf 522 sequence from sunflower (Helianthus annuus) and bordered by the primers of the sequences SEQ ID No. 1 and SEQ ID No. 2 or a sequence with at least 90% homology with said 347 bp nucleotide sequence which is derived from the orf 522 sequence and bordered by the primers of the sequences SEQ ID No. 1 and SEQ ID No. 2.

2. Mitochondrion comprising a recombinant genome according to Claim 1.

3. Plant cytoplasm comprising the genome of the genus Cichorium and mitochondria according to Claim 2.

4. Recombinant plant cell comprising a cytoplasm according to Claim 3.

5. Method of producing a plant of the Cichorium genus which exhibits cytoplasmic male sterility, **characterized in that** a 347 bp nucleotide sequence which is derived from the orf 522 sequence from sunflower (Helianthus annuus) and bordered by the primers of the sequences SEQ ID No. 1 and SEQ ID No. 2 or a sequence with at least 90% homology with said 347 bp nucleotide sequence which is derived from the orf 522 sequence and bordered by the primers of the sequences SEQ ID No. 1 and SEQ ID No . 2 is integrated into the cellular genome of the said plant.

6. Use of the 347 bp nucleotide sequence which is derived from the orf 522 sequence from sunflower (Helianthus annuus) and bordered by the primers of the sequences SEQ ID No. 1 and SEQ ID No. 2 or a sequence with at least 90% homology with said 347 bp nucleotide sequence which is derived from the orf 522 sequence and bordered by the primers of the sequences SEQ ID No. 1 and SEQ ID No. 2 to confer cytoplasmic male sterility to a plant of the genus Cichorium.

7. Method of selecting cytoplasmic male sterility in a plant of the genus Cichorium, **characterized in that** the mitochondrial nucleic acid of the plant is brought into contact with a labeled probe comprising at least 10 consecutive nucleotides of the 347 bp nucleotide sequence which is derived from the orf 522 from sunflower (Helianthus annuus) and bordered by the primers of the sequences SEQ ID No. 1 and SEQ ID No.2.

## Patentansprüche

1. Rekombinantes pflanzliches Genom der Gattung Cichorium, **dadurch gekennzeichnet, dass** es für die Zichorien spezifische Gene und eine Nukleotidsequenz von 347 bp, welche von der Sequenz des orf 522 von Sonnenblume (Helianthus annuus) abgeleitet ist und durch die Primer mit den Sequenzen SEQ ID NO: 1 und SEQ ID NO: 2 begrenzt wird, oder eine Sequenz, welche wenigstens 90% Homologie zu dieser Nukleotidsequenz von 347 bp, welche von der Sequenz des orf 522 abgeleitet ist und durch die Primer mit den Sequenzen SEQ ID NO: 1 und 2 begrenzt wird, aufweist, umfasst.

2. Mitochondrion, welches ein rekombinantes Genom nach Anspruch 1 umfasst.

3. Pflanzliches Zytoplasma, welches das Genom der Gattung Cichorium und Mitochondrien nach Anspruch 2 umfasst.

4. Rekombinante pflanzliche Zelle, welche ein Zytoplasma nach Anspruch 3 umfasst.

5. Verfahren zur Herstellung einer Pflanze der Gattung Cichorium, welche eine zytoplasmatische männliche Sterilität aufweist, **dadurch gekennzeichnet, dass** man in das zelluläre Genom dieser Pflanze eine Nukleotidsequenz von 347 bp, welche von der Sequenz des orf 522 von Sonnenblume (Helianthus annuus) abgeleitet ist und durch die Primer mit den Sequenzen SEQ ID NO: 1 und SEQ ID NO: 2 begrenzt wird, oder eine Sequenz, welche wenigstens 90% Homologie zu dieser Nukleotidsequenz von 347 bp, welche von der Sequenz des orf 522 abgeleitet ist und durch die Primer mit den Sequenzen SEQ ID NO: 1 und 2 begrenzt wird, aufweist, integriert.

6. Verwendung der Nukleotidsequenz von 347 bp, welche von der Sequenz des orf 522 von Sonnenblume (Helianthus annuus) abgeleitet ist und durch die Primer mit den Sequenzen SEQ ID NO: 1 und SEQ ID NO: 2 begrenzt wird, oder einer Sequenz, welche wenigstens 90% Homologie zu dieser Nukleotidsequenz von 347 bp, welche von der Sequenz des orf 522 abgeleitet ist und durch die Primer mit den Sequenzen SEQ ID NO: 1 und 2 begrenzt wird, aufweist, um einer Pflanze der Gattung Cichorium eine zytoplasmatische männliche Sterilität zu verleihen.

7. Verfahren zur Selektion einer zytoplasmatischen männlichen Sterilität bei einer Pflanze der Gattung Cichorium, **dadurch gekennzeichnet, dass** man die mitochondriale Nukleinsäure der Pflanze mit einer markierten Sonde, umfassend wenigstens 10 aufeinander folgende Nukleotide der Nukleotidsequenz von 347 bp, welche von der Sequenz des orf 522 von Sonnenblume (Helianthus annuus) abgeleitet ist und durch die Primer mit den Sequenzen SEQ ID NO: 1 und SEQ ID NO: 2 begrenzt wird, in Kontakt bringt.
